Europäisches Patentamt

European Patent Office

Office européen des brevets

(11) Publication number: **0 351 944**
**A1**

## EUROPEAN PATENT APPLICATION

(21) Application number: 89305174.8

(22) Date of filing: 23.05.89

(51) Int. Cl.4: **C07K 7/08 , C12P 21/00 , G01N 33/68**

(30) Priority: 26.05.88 US 199110

(43) Date of publication of application:
**24.01.90 Bulletin 90/04**

(84) Designated Contracting States:
**ES GR**

(71) Applicant: **Schering Biotech Corporation**
**901 California Avenue**
**Palo Alto California 94304-1104(US)**

(72) Inventor: **Abrams, John S.**
**1817 Oak Knoll Drive**
**Belmont California 94002(US)**
Inventor: **Pearce, Michael K.**
**3375 Alma Avenue Apt. 377**
**Palo Alto California 94301(US)**

(74) Representative: **Ritter, Stephen David et al**
**Mathys & Squire 10 Fleet Street**
**London EC4Y 1AY(GB)**

(54) **Immunogenic human interleukin-3 peptide and monoclonal antibodies thereto.**

(57) An immunogenic human interleukin-3 (IL-3) peptide is provided from which monoclonal peptide antibodies can be produced. The peptide of the invention corresponds to an interior subsequence of the IL-3 protein. The invention also includes conjugates of the immunogenic peptide and carriers, and monoclonal antibodies specific for the immunogenic peptide.

EP 0 351 944 A1

## IMMUNOGENIC HUMAN INTERLEUKIN-3 PEPTIDE AND MONOCLONAL ANTIBODIES THERETO

### Field of the Invention

The invention relates generally to peptides useful in the generation of antibodies, and more particularly to an immunogenic peptide of human interleukin-3 and monoclonal antibodies specific therefor.

### Background

Circulating blood cells are constantly replaced by newly developed cells. Replacement blood cells are formed in a process termed hematopoiesis which involves the production of at least eight mature blood cell lineages: red blood cells (erythrocytes), macrophages (monocytes), eosinophilic granulocytes, megakaryocytes (platelets), neutrophilic granulocytes, basophilic granulocytes (mast cells), T lymphocytes, and B lymphocytes: Burgess and Nicola, Growth Factors and Stem Cells (Academic Press, New York, 1983). Much of the control of blood cell formation is mediated by a group of interacting glycoproteins termed colony stimulating factors (CSFs). These glycoproteins are so named because of the in vivo and in vitro assays used to detect their presence. Techniques for the clonal culture of hematopoietic cells in semisolid culture medium have been especially important in the development of in vitro assays. In such cultures, individual progenitor cells (i.e., cells developmentally committed to a particular lineage, but still capable of proliferation) are able to proliferate to form a colony of maturing progeny in a manner which is believed to be essentially identical to the comparable process in vivo. The role of CSFs in hematopoiesis is the subject of many recent reviews, e.g.: Metcalf, The Hemopoietic Colony Stimulating Factors (Elsevier, New York, 1984); Metcalf, Proc. R. Soc. Lond. B, Vol. 230, pgs. 389-423 (1987); Clark et al., Science, Vol. 236, pgs. 1229-1237 (1987); and Sachs, Science, Vol. 238, pgs. 1374-1379 (1987).

The detection, isolation and purification of these factors is extremely difficult, for it is frequently complicated by the nature of the supernatants they are typically located in, the presence of parallel-acting factors within the mixtures, the sensitivity (or lack thereof) of the assays utilized to ascertain the factors' properties, the frequent similarity in the range of molecular weights and other characteristics of the factors, and the very low concentration of the factors in their natural settings.

As more CSFs become available, primarily through molecular cloning, interest has heightened in finding clinical applications for them. Because of physiological similarities to hormones (e.g., soluble factors, growth mediators, action via cell receptors), potential uses of CSFs have been analogized to the current uses of hormones: e.g. Dexter, Nature, Vol. 321, pg. 198 (1986). Their use has been suggested for several clinical situations where the stimulation of blood cell generation would be desirable, such as for rehabilitative therapy after chemotherapy or radiation therapy of tumors, treatment of myeloid hypoplasias, treatment of neutrophil deficiency, treatment to enhance hematopoietic regeneration following bond marrow transplantation, and treatment to increase host resistance to established infections, e.g. Dexter (cited above).

Over the past several years a murine CSF activity, termed multi-CSF or interleukin-3 (IL-3), has been identified, purified, and cloned: Yokota et al.; Proc. Natl. Acad. Sci., Vol. 81, pgs. 1070-1074 (1984); Fung et al., Nature, Vol. 307, pgs. 233-237 (1984); Hapel et al., Blood, Vol. 65, pgs. 1453-1459 (1985); Ihle et al., J. Immunol., Vol. 129, pgs. 2431-2436 (1982); Ihle et al., J. Immunol., Vol. 131, pgs. 282-287 (1983); and Iscove et al., pgs. 397-425 in Cellular and Molecular Biology of Lymphokines - (Academic Press, New York, 1985). Murine IL-3 exhibits a broad spectrum of CSF activities, for it is capable of stimulating proliferation and development of the progenitor cells of all the recognized myeloid lineages, e.g.: Dexter, Nature, Vol. 309, pgs. 746-747 (1984); Ihle et al., Lymphokines, Vol. 9, pgs. 153-200 (1984). Recently, rat, gibbon, and human homologues of mouse IL-3 have been reported, and appear to have the same broad spectrum of activities as the murine counterpart: Cohen et al., Nucleic Acids Research, Vol. 14, pgs. 3641-3658 (1986); Yang et al., Cell, Vol. 47, pgs. 3-10 (1986), and Otsuka, J. Immunol., Vol. 140, pgs 2288-2295 (1988).

The development of monoclonal antibodies specific for proteins of therapeutic interest has been an important step towards the development of efficient immunopurification protocols and diagnostic kits, e.g.: Secher et al., Nature, vol. 285, pgs. 426-430 (1980); Secher et al., U.S. patent 4,423,147; and Bartholomew et al., PCT publication WO 83/03678. Frequently, the development of peptide antibodies is the most expedient manner of obtaining monoclonal antibodies specific for a protein of interest, particularly when the protein's gene has been cloned and sequenced, but sufficient quantities of the purified protein are not yet available for immunizations, e.g.: Walter et al., Genetic Engineering, Vol. 5, pgs. 61-91 (1983); Lerner et al., Proc. Natl. Acad. Sci., Vol. 78, pgs. 3403-3407 (1981); and Bulinski, Internatl. Rev. Cytol., Vol. 103, pgs. 281-302 (1986). A major problem of this approach is how to select from the deduced amino acid sequence a peptide which corresponds to an antigenic determinant of the protein of interest. A large number of factors apparently determine which sequences of amino acids within a protein make up its antigenic determinants, and at present only a handful of imperfect criteria are available for correlating particular amino acid sequences with antigenic determinants, e.g.: Hopp et al., Proc. Natl. Acad. Sci., Vol. 76, pgs. 3824-3828 (1981); Westof et al., Nature, Vol. 311, pgs. 123-126 (1984); Fraga, Can. 3. Chem., Vol. 60, pgs. 2606-2610 (1982); Novotny et al., Proc. Natl. Acad. Sci., Vol. 83, pgs. 226-230 (1986); Berkofsky, Science, Vol. 229, pgs. 932-940 (1985); Tainer et al., Nature, Vol. 312, pgs. 127-134 (1984); Palfreyman et al., J. Immunol. Meth., Vol. 75, pgs. 383-393 (1984).

In view of the potential clinical utility of human interleukin-3 proteins, the development of monclonal antibodies for purification and detection would be highly desirable.

## SUMMARY OF THE INVENTION

The invention is directed to the human interleukin-3 peptide defined by the following formula I:
H-PNLEAFNRAVKSLQNASAI-OH
(alternatively:
H-Pro-Asn-Leu-Glu-Ala-Phe-Asn-Arg-Ala-Val-Lys-Ser-Leu-Gln-Asn-Ala-Ser-Ala-Ile-OH),
immunogens comprising conjugates between a carrier and the above peptide, and monoclonal antibodies specific for the above peptide. The term "immunogen" as used herein refers to a substance which is capable of causing an immune response. The term "carrier" as used herein refers to any substance which when chemically conjugated to a peptide of the invention permits a host organism immunized with the resulting conjugate to generate antibodies specific for the conjugated peptide. Carriers include red blood cells, bacteriophages, proteins, or synthetic particles such as agarose beads. Preferably carriers are proteins, such as serum albumin, gamma-globulin, keyhole limpet hemocyanin, throglobulin, ovalbumin, fibrinogen, myoglobin, or the like.

Standard symbols are used throughout for amino acids, e.g. Cohen, "Nomenclature and Symbolism of alpha-Amino Acids", Methods in Enzymology, Vol. 106, pgs. 3-17 (Academic Press, N.Y.,

1984). Accordingly, Table I of that reference is incorporated herein by reference.

## DETAILED DESCRIPTION OF THE INVENTION

The peptide of the invention can be synthesized by standard techniques, e.g. Stewart and Young, Solid Phase Peptide Synthesis, 2nd Ed. (Pierce Chemical Company, Rockford, IL, 1984). Preferably a commercial automated synthesizer is used, e.g. Vega Biochemicals (Tucson, AZ) models 296A or B, or Applied Biosystems, Inc. (Foster City, CA) model 430A.

The peptide of the invention can be assembled by solid phase synthesis on a cross-linked polystyrene support starting from the carboxyl terminal residue and adding amino acids in a stepwise fashion until the entire 19-residue chain has been formed. We performed synthesis on a fully automated peptide synthesizer (Applied Biosystems, Inc. model 430A). The following references are guides to the chemistry employed during synthesis: Merrifield, J. Amer. Chem. Soc., Vol. 85, pg. 2149 (1963); Kent et al., pg. 185, in Peptides 1984, Ragnarsson, Ed. (Almquist and Weksell, Stockholm, 1984); Kent et al., pg. 217 in Peptide Chemistry 84, Izumiya, Ed. (Protein Research Foundation, B.H. Osaka, 1985); Merrifield, Science, Vol. 232, pgs. 341-347 (1986); and references cited in this last reference.

In solid state synthesis it is most important to eliminate synthetic by-products, which are primarily termination, deletion, or modification peptides. Most side reactions can be eliminated or minimized by use of clean, well-characterized resins, clean amino acid derivatives, clean solvents, and the selection of proper coupling and cleavage methods and reaction conditions; e.g. Barany and Merrifield, The Peptides, Cross and Meienhofer, Eds., Vol. 2, pgs. 1-284 (Academic Press, New York, 1979). It is important to monitor coupling reactions to determine that they proceed to completion so that deletion peptides lacking one or more residues will be avoided. The quantitative ninhydrin reaction is useful for that purpose; Sarin et al., Anal. Biochem., Vol. 117, pg. 147 (1981). Na-t-butyloxycarbonyl-amino acids were used with appropriate side-chain protecting groups stable to the conditions of chain assembly but labile to strong acids. After assembly of the protected peptide chain, the protecting groups were removed and the peptide-anchoring bond was cleaved by the use of low and then high concentrations of anhydrous hydrogen fluoride in the presence of a thioester scavenger: Tam et al., J. Amer. Chem. Soc., Vol. 105, pg. 6442 (1983).

Side-chain protecting groups used were Asp-

(OBzl), Glu(OBzl), Ser(Bzl), Thr(Bzl), Lys(Cl-Z), Tyr (Br-Z), Arg($N^G$Tos), Cys(4-MeBzl), and His(ImDNP). (Bzl = benzyl; Tos = toluene sulfonyl; DNP = dinitrophenyl; Im = imidazole; 2 = benzyloxycarbonyl.) The remaining amino acids had no protecting groups on their side-chains. All the amino acids were obtained from Peninsula Laboratories, except the tBoc-His (ImDNP), which was from Chemical Dynamics and was recrystallized from ethanol before use. [t-Boc = N2-t-butyloxycarbonyl.] For each cycle the tBoc-Na-protected peptide-resin was exposed to 65 percent trifluoroacetic acid (from Eastman Kodak) (distilled before use) in dichloromethane (DCM), (Mallenckrodt): first for 1 minute and then for 13 minutes to remove the Na-protecting group. The peptide-resin was washed with DCM and then neutralized twice with 10 percent diisopropylethylamine (DIEA) (Aldrich) in dimethylformamide (DMF) (Applied Biosystems), for 1 minute each. Neutralization was followed by washing with DMF. Coupling was performed with the preformed symmetric anhydride of the amino acid in DMF for 16 minutes. The preformed symmetric anhydride was prepared on the synthesizer by dissolving 2 mmol of amino acid in 6 ml of DCM and adding 1 mmol of dicyclohexycarbodiimide (Aldrich) in 2 ml of DCM. After 5 minutes, the activated amino acid was transferred to a separate vessel and the DCM was evaporated by purging with a continuous stream of nitrogen gas. The DCM was replaced by DMF (6 ml total) at various stages during the purging. After the first coupling, the peptide-resin was washed with DCM, 10 percent DIEA in DCM, and then with DCM. For recoupling, the same amino acid and the activating agent, dicyclohexylcarbodiimide, were transferred sequentially to the reaction vessel. After activation in situ and coupling for 10 minutes, sufficient DMF was added to make a 50 percent DMF-DCM mixture, and the coupling was continued for 15 minutes. Arginine was coupled as a preformed ester with hydroxybenzotriazole (Aldrich) in DMF for 60 minutes and then recoupled in the same manner as the other amino acids. Asparagine and glutamine were coupled twice as preformed hydroxybenzotriazole esters in DMF: 40 minutes for each coupling. For all residues, the resin was washed after the second coupling and a sample was automatically taken for monitoring residual uncoupled a-amine by quantitative ninhydrin reaction (Sarin et al., cited above).

The general technique of linking synthetic peptides to a carrier is described in several references, e.g.: Walter and Doolittle, "Antibodies Against Synthetic Peptides," in Genetic Engineering (Setlow et al., eds.), Vol. 5, pgs. 61-91 (Plenum Press, N.Y., 1983); Green et al., Cell, Vol. 28, pgs. 477-487 (1982); Lerner et al., Proc. Natl. Acad. Sci., Vol. 78, pgs. 3403-3407 (1981); Shimizu et al., U.S. Patent 4,474,754; and Ganfield et al., U.S. Patent 4,311,639. Accordingly, these references are incorporated by reference. Also, techniques employed to link haptens to carriers are essentially the same as the above-referenced techniques; e.g. chapter 20 in Tijssen Practice and Theory of Enzyme Immunoassays (Elsevier, New York, 1985)

The four most commonly used methods for attaching a peptide to a carrier use (1) glutaraldehyde for amino coupling, e.g. as disclosed by Kagan and Glick, in Jaffe and Behrman, eds. Methods of Hormone Radioimmunoassay, pgs. 328-329 (Academic Press, N.Y. 1979), and Walter et al., Proc. Natl. Acad. Sci., Vol. 77, pgs. 5197-5200 (1980); (2) water-soluble carbodiimides for carboxyl-to-amino coupling, e.g. as disclosed by Hoare et al., J. Biol. Chem., Vol. 242, pgs. 2447-2453 (1967); (3) bis-diazobenzidine (BDB) for tyrosine-to-tyrosine sidechain coupling, e.g. as disclosed by Bassiri et al., pgs. 46-47, in Methods of Hormone Radioimmunoassay, Jaffe and Behrman, eds. (cited above), and by Walter et al. (cited above); and (4) maleimidobenzoyl-N-hydroxysuccinimide ester (MBS) for coupling cysteine (or other sulfhydryls) to amino groups, e.g. as disclosed by Kitagawa et al., J. Biochem. (Tokyo), Vol. 79, pgs. 233-239 (1976), and by Lerner et al. (cited above).

A general rule for selecting an appropriate method for coupling a given peptide to a protein carrier can be stated as follows: the amino acid chosen for coupling should occur only once in the sequence, preferably at the appropriate end of the segment. For example, BDB should not be used if a tyrosine residue occurs in the main part of a sequence chosen for its potentially antigenic character. Similarly, centrally located lysines rule out the glutaraldehyde method, and the occurrence of aspartic or glutamic acid will frequently exclude the carbodiimide method. On the other hand, suitable amino acid residues can be positioned at either end of a chosen sequence segment as attachment sites, whether or not they naturally occur there in the chosen protein sequence.

If the selected immunogenic peptide fragment does not extend to the carboxyl or amino terminus of IL-3, then its unattached end may present a terminus that does not occur in IL-3; consequently the immunogenic peptide fragment will differ significantly at that terminus from the native IL-3. Any problems caused by this difference can be obviated to some extent if the carboxy terminus is chosen for attachment to the protein carrier and the free amino end is acetylated.

The coupling efficiency of the peptide of formula I to the carrier protein is conveniently measured by using a radioactively labeled peptide, prepared either by using a radioactive amino acid for one step of the synthesis or by labeling the completed peptide by the iodination of a tyrosine residue. The presence of tyrosine in the peptide also allows one to set up a sensitive radioimmunoassay, if desirable. Therefore, it may be desirable to introduce tyrosine as a terminal residue if it is not part of the peptide sequence of the invention.

Preferred carriers are proteins, and preferred protein carriers include bovine serum albumin, myoglobulin, ovalbumin (OVA), keyhole limpet hemocyanin (KLH), or the like.

Peptides can be linked to KLH through cysteines by MBS as disclosed by Liu et al., Biochemistry, Vol. 18, pgs. 690-697 (1979). The peptides are dissolved in phosphate-buffered saline (pH 7.5), 0.1M sodium borate buffer (pH 9.0) or 1.0M sodium acetate buffer (pH 4.0). The pH for the dissolution of the peptide is chosen to optimize peptide solubility. The content of free cysteine for soluble peptides is determined by the method of Ellman, Arch. Biochem. Biophys., Vol. 82, pg. 7077 (1959).

For each peptide, 4 mg KLH in 0.25 ml of 10 mM sodium phosphate buffer (pH 7.2) is reacted with 0.7 mg MBS (dissolved in dimethyl formamide) and stirred for 30 min. at room temperature. The MBS is added dropwise to ensure that the local concentration of formamide is not too high, as KLH is insoluble in J30% formamide. The reaction product, KLH-MB, is then passed through Sephadex G-25 equilibrated with 50 mM sodium phosphate buffer (pH 6.0) to remove free MBS; KLH recovery from peak fractions of the column eluate (monitored by $OD_{280}$) is estimated to be approximately 80%.

KLH-MB is then reacted with 5 mg peptide dissolved in 1 ml of the chosen buffer. The pH is adjusted to 7-7.5 and the reaction is stirred for 3 hr at room temperature. Coupling efficiency is monitored with radioactive peptide by dialysis of a sample of the conjugate against phosphate-buffered saline; it typically ranges from 8% to 60%.

Once the peptide-carrier conjugate is available, polyclonal or monoclonal antibodies are produced by standard techniques, e g.: as disclosed by Campbell in Monoclonal Antibody Technology - (Elsevier, New York, 1984); Hurrell, ed., Monoclonal Hybridoma Antibodies: Techniques and Applications (CRC Press, Boca Raton, FL, 1982); Schreier et al. Hybridoma Techniques (Cold Spring Harbor Laboratory, New York, 1980); U.S. Patent 4,562,003; or the like. In particular, U.S. Patent 4,562,003 is incorporated by reference. For monoclonal antibody production, the first step is to immunize a host animal to obtain a source of B lymphocytes. The B lymphocytes are fused with an appropriate immortalizing cell line to form hybridomas that secrete monoclonal antibodies. Immortalizing cell lines are usually tumor cell lines, such as myelomas. Preferably, the host animals are rodents, and the immortalizing cell line also is preferably derived from rodent cells, especially from the same rodent species. After formation, hybridomas are screened for those producing antibodies against the peptide of the invention. Immunization, harvesting of lymphocytes, and cell fusion are all techniques well known in the art. Immunization is carried out by a regimen of repeated injections into the host animal of the purified peptide-carrier conjugate, usually mixed with a suitable adjuvant. Immunization can be optimized by varying several factors, including the amount of antigen used for the primary injection and subsequent boosts, the route of injection, the time schedule for injecting and bleeding, and the use of adjuvant, e.g. Freund's complete or incomplete adjuvant. Techniques for fusion are also well known in the art, and in general involve mixing the cells with a fusing agent such as, most commonly, polyethylene glycol.

Successful hybridoma formation is assessed and selected by standard procedures such as, for example, HAT selection. From among proliferating hybridomas, those successfully secreting the desired antibody are selected by assaying the culture medium for their presence. Ordinarily this is done using immunoreaction-based assays, for example Western blot, ELISA, or RIA assays. The antibodies can be recovered from the medium using standard protein purification techniques.

Both polyclonal and monoclonal antibodies can be screened by ELISA. As in other solid phase immunoassays, the test is based on the tendency of macromolecules to adsorb nonspecifically to plastic. The irreversibility of this reaction, without loss of immunological activity, allows the formation of antigen-antibody complexes with a simple separation of such complexes from unbound material.

To titrate antipeptide serum, peptide conjugated to a carrier different from that used in immunization, or free peptide alone, is adsorbed to the wells of a 96-well microtiter plate. The adsorbed antigen is then allowed to react in the wells with dilutions of anti-peptide serum. Unbound antibody is washed away, and the remaining antigen-antibody complexes are allowed to react with antibody specific for the IgG of the immunized animal; this second antibody is conjugated to an enzyme such as alkaline phosphatase. A visible colored reaction product produced when the enzyme substrate is added indicates which wells have bound antipeptide antibodies. The use of a spectrophotometer allows better quantification of the amount of peptide-specific antibody bound. High-titer antisera yield a linear titration curve between $10^{-3}$ and $10^{-5}$ dilutions.

## Examples

The following examples serve to illustrate the present invention. Selection of particular peptides and/or carriers, and the choice of temperatures, concentrations, and the values of other variables are only to exemplify application of the present invention and are not to be considered limitations therof.

## EXAMPLE 1

### Coupling (C) PNLEAFNRAVKSLQNASAI to Ovalbumin and/or Myoglobin and Production of Polyclonal Antibodies Thereto

(C) indicates a cysteine bridge to the carrier protein.

50 mg of ovalbumin (OVA) and 50 mg of myoglobulin (MYO) (e.g. available from Sigma) were each dissolved in 10 ml of 0.1M sodium bicarbonate, and reacted with 1 ml of 0.12 iodoacetamide solution (88 mg of iodoacetamide dissolved in 4 ml 0.1M sodium bicarbonate) for 1 hour at room temperature in a 15 ml Falcon tube (Falcon Plastics, Oxnard, CA), or the like. Each reaction mixture was dialyzed overnight against 4 liters of 0.1M sodium bicarbonate at 4°C. Separately, 10 mg of (C)PNLEAFNRAVKSLQNASAI was dissolved in 2 ml of 0.1M DTT (dithiothreitol) solution (containing 50 mM Tris and 2.5 mM EDTA at pH 8) in a 4 ml tube, incubated at 37°C overnight, and then applied to a GFO5 gel-filtration column (1.5 x 26.5 cm) (LKB, Bromma, Sweden) and eluted with a peptide elution buffer consisting of 0.015M acetic acid and 0.005 M beta-mercaptoethanol. Three fractions of about 3.5 ml each which contained the reduced peptide were identified by optical density at 206 nm, collected, pooled, frozen in dry ice, and lyophilized overnight. Meanwhile OVA and MYO were recovered from dialysis, and clarified by filtration through 0.45 micrometer filters. OVA and MYO were activated by mixing each with 380 microliters of N-hydroxysuccinimide ester of iodoacetic acid (NHIA) (disclosed by Rector et al., in J. Immunol. Meth., Vol. 24, pg. 321 (1978)) dissolved in tetrahydrofuran (5 mg/ml), stirring for 30 minutes at room temperature, and dialyzing overnight against 4 liters PBS (1.8g

$NaH_2PO_4 \bullet H_2O$, 7.2 g $Na_2HPO_4 \bullet 7H_2O$; and 34 g NaCl in 4 liters water). Separately the lyophilized peptide was resuspended in 5 ml of borate reduction buffer (2g $Na_2B_4O_7 \bullet 10H_2O$, 17.4g NaCl, and 336 mg $EDTA \bullet Na_2$ in one liter water with pH adjusted to 8.5 with concentrated HCl, deoxygenated under nitrogen for 15 minutes, after which 178 mg ascorbate is added). The dialyzed iodoacetylated OVA and MYO were recovered, separately mixed with equal volumes (preferably 2 ml) of borate reduction buffer containing the peptide, and incubated overnight at room temperature. The resulting conjugates were analyzed by SDS-PAGE (12.5% gel). The conjugate-containing solution was diluted with PBS to 1 mg/ml, filtered sterile, and aliquots of convenient volumes (e.g. 500 microliters) were taken for immunizations, and/or stored at 4°C.

Polyclonal antisera against the MYO conjugate were produced in rats. The immunization schedule was as follows: Initially, 100 )g of the MYO conjugate in 0.5 ml PBS was mixed with 0.5 ml of Freund's Complete Adjuvant (FCA) and injected i.p. into the rat. Additionally, i.p. injections of identical composition were given on days 17 and 86, after which a positive reaction against PNLEAFNRAVKSLQNASAI was detected by ELISA. The rat polyclonal anti-serum was also used in a Western blot analysis of electrophoretically separated recombinant human IL-3 (Otsuka et al., cited above). A commercially-obtained sheep anti-rat immunoglobulin reagent was used as the second-stage label.

Supernatant of COS7 cells transfected with pcD-SRa-IL-3 and medium from stationary phase cultures of yeast harboring phIL-3-14 were concentrated by using Centricon 10 concentrators (Amicon Corp., Davers, MA). The recovered sample was diluted 1/1 with SDS sample buffer containing 0.062M Tris-HCl (pH 6.8), 2% SDS, 10% glycerol, and 5% 2-ME, and boiled for 5 min. The samples were loaded on an SDS-15% polyacrylamide gel with a discontinuous buffer system (Laemli, Nature, Vol. 227, pg. 680 (1970)). After electrophoresis, the protein was transferred electrophoretically to a nitrocellulose membrane overnight at 0.2 A in 20 mM Tris base, 150 mM glycine and 20% methanol, at 4°C. The membrane was blocked in 100 ml of 0.5% BSA in PBS. The first stage antibodies were dilutions of the rat antisera against human IL-3 in PBS containing 0.1% BSA and 0.05% Tween 20. The membrane was incubated in 50 ml of this solution for 2 hours and then washed in three changes of PBS-BSA-Tween buffer for 20 min. each. A 50-)1 volume of [125]-I-labeled sheep anti-rat Ig in 50 ml of PBS-BSA-Tween was used as the second-stage labeled antibody. The blots were incubated for 2 hours and then washed again as described above. They were then dried briefly and exposed to X-ray film. The human recombinant IL-3 band was successfully identified by the antiserum.


EXAMPLE II


Production of Hybridoma MP3.8A5.12


The rat of Example I was given a final i.p. injection and an i.v. injection of identical composition to the prior injections (200 )g conjugate in all) on day 135. Four days later the rat was sacrificed and spleen cells were fused with mouse myeloma cells, P2X63-Ag8.653 (ATCC CRL 1580), in a 1:1 ratio using polyethylene glycol (PEG). The cell suspension ($3.5 \times 10^{-5}$ cells/ml) in HAT medium was distributed into 40 96-well plates. Ten days later hybridoma supernatants were tested for their ability to bind to human IL-3 immobilized directly on microtiter plates (indirect ELISA). Bound antibody was detected by peroxidase conjugated goat anti-rat immunoglobulin with a standard protocol. Hybridomas secreting antibodies reacting with IL-3 were cloned by limiting dilution. MP3.8A5.12 was one such hybridoma selected by these procedures. Antibodies from MP3.8A5.12 were ·determined to be of the $IgG_{2a}$ isotype. The hybridoma can be stored (e.g. -70`C in culture medium with 10% DMSO) and cultured using standard mammalian cell culture techniques (e.g., RPMI 1640 with 10% fetal bovine serum, supplemented with 1 mM glutamine and 50 mM 2-mercaptoethanol)

The invention further provides a kit for detecting the presence of human interleukin-3 in a sample suspected of containing human interleukin-3, the kit comprising:
a first monoclonal antibody specific for a first antigenic determinant on human interleukin-3;
a second antibody selected from the group consisting of a polyclonal antibody composition specific for human interleukin-3 and a second monoclonal antibody specific for a second antigenic determinant on human interleukin-3, the second antigenic determinant being different from the first antigenic determinant;
a support means; and
a signal-generating means.

In this kit, the first monoclonal antibody can be the monoclonal antibody produced by hybridoma MP3.8A5.12 and the second antibody can be a polyclonal antibody composition specific for human interleukin-3. The signal-generating means can comprise an enzyme operationally associated with said first monoclonal antibody, the enzyme being selected from the group consisting of peroxidase, beta-galactosidase, and alkaline phosphatase. The general principles for the presentation of such kits are well known.

The descriptions of the foregoing embodiments of the invention have been presented for the purpose of illustration and description. They are not intended to be exhaustive or to limit the invention to the precise form disclosed, and obviously many modifications and variations are possible in light of the above teaching. The embodiments were chosen and described in order to best explain the principles of the invention and its practical application to thereby enable others skilled in the art to best utilize the invention in various embodiments and with various modifications as are suited to the particular use contemplated. It is intended that the scope of the invention be defined by the claims appended hereto.

Applicants have deposited hybridoma MP3.8A5.12 with the American Type Culture Collection, Rockville, MD, USA (ATCC), under accession number HB 9727. This deposit was made under conditions as provided under ATCC's agreement for Culture Deposit for Patent Purposes, which assures that the deposit will be made available to the US Commissioner of Patents and Trademarks pursuant to 35 USC 122 and 37 CFR 1.14, and will be made available to the public upon issue of a U.S. patent, which requires that the deposit be maintained. Availability of the deposited strain is not to be construed as a license to practice the invention in contravention of the rights granted under the authority of any government in accordance with its patent laws.

The deposit has been modified to conform to the requirements of the Budapest Treaty on the Deposit of Microorganisms.


**Claims**


1. An immunogenic human interleukin-3 peptide defined by the amino acid sequence:
H-PNLEAFNRAVKSLQNASAI-OH.

2. A compound comprising the peptide
H-PNLEAFNRAVKSLQNASAI-OH
conjugated to a carrier.

3. The compound of claim 2 wherein said carrier is selected from the group consisting of ovalbumin, myoglobulin, keyhole limpet hemocyanin, thyroglobulin, bovine serum albumin, and fibrinogen.

4. A monoclonal peptide antibody specific for the peptide
H-PNLEAFNRAVKSLQNASAI-OH.

5. The monoclonal antibody of claim 4 produced by the hybridoma MP3.8A5.12.

6. The hybridoma MP3.8A5.12.

7. A kit for detecting the presence of human interleukin-3 in a sample suspected of containing human interleukin-3, the kit comprising:
a first monoclonal antibody specific for a first antigenic determinant on human interleukin-3;
a second antibody selected from the group consisting of a polyclonal antibody composition specific for human interleukin-3 and a second monoclonal antibody specific for a second antigenic determinant on human interleukin-3, the second antigenic determinant being different from the first antigenic determinant;
a support means; and
a signal-generating means.

8. The kit of claim 7 wherein said first monoclonal antibody can be the monoclonal antibody produced by hybridoma MP3.8A5.12 and wherein said second antibody can be a polyclonal antibody composition specific for human interleukin-3.

9. The kit of claim 8 wherein said signal generating means can comprise an enzyme operationally associated with said first monoclonal antibody, the enzyme being selected from the group consisting of peroxidase, beta-galactosidase, and alkaline phosphatase.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | SCIENCE, vol. 231, 10th January 1986, pages 134-139; I. CLARK-LEWIS et al.: "Automated chemical synthesis of a protein growth factor for hemopoietic cells, interleukin-3" * Whole article * | 1,4 | C 07 K 7/08 C 12 P 21/00 G 01 N 33/68 |
| A | THE JOURNAL OF IMMUNOLOGY, vol. 140, no. 4, 15th February 1988, pages 1182-1187, The American Association of Immunologists, US; H.J. ZILTENER et al.: "Monoclonal antipeptide antibodies recognize IL-3 and neutralize its bioactivity in vivo" * Whole article * | 1,4 | |

TECHNICAL FIELDS SEARCHED (Int. Cl.4)

C 07 K
C 12 P

The present search report has been drawn up for all claims

| Place of search | Date of completion of the search | Examiner |
|---|---|---|
| THE HAGUE | 04-09-1989 | RAJIC M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)